# EUROPEAN PATENT SPECIFICATION

(11) **EP 1 303 269 B1**
(45) Date of publication and mention of the grant of the patent: **07.12.2005**
(21) Application number: 01940246.0
(22) Date of filing: 14.06.2001
(51) Int. Cl.: A61K 31/405, A61P 43/00, A61P 25/00

(54) **USE OF 3-SUBSTITUTED OXINDOLE DERIVATIVES AS KCNQ POTASSIUM CHANNEL MODULATORS**
VERWENDUNG VON 3-SUBSTITUIERTEN OXIDINOLDERIVATEN ALS KCNQ KALIUMKANALMODULATOREN
UTILISATION DE DERIVES D'OXINDOLE 3-SUBSTITUE COMME MODULATEURS DU CANAL POTASSIQUE KCNQ

(30) Priority: 29.06.2000 DK 200001022; 08.03.2001 DK 200100394
(43) Date of publication of application: 23.04.2003
(73) Proprietor: NEUROSEARCH A/S, 2750 Ballerup (DK)
(72) Inventor: JENSEN, Bo, Skaaning, c/o NeuroSearch A/S, DK-2750 Ballerup (DK); SCHROEDER, Rikke, L., DK-2000 Frederiksberg (DK); STROEBAEK, Dorte, c/o NeuroSearch A/S, DK-2750 Ballerup (DK); OLESEN, Soeren, Peter, c/o NeuroSearch A/S, DK-2750 Ballerup (DK)
(74) Representative: Halberg, Kristian
(86) International application number: PCT/DK2001/000412
(87) International publication number: WO 2002/000217

(56) References cited:
- EP-A- 0 747 354
- WO-A-00/44786
- WO-A-01/10381
- WO-A-98/16222
- RIKKE LOUISE SCHRÖDER ET AL: "KCNQ4 channel activation by BMS-204352 and retigabine" NEUROPHARMACOLOGY, vol. 40, no. 7, June 2001 (2001-06), pages 888-898, XP002901941
- VALENTIN K. GRIBKOFF ET AL: "Targeting acute ischemic stroke with a calcium-sensitive opener of maxi-K potassium channels " NATURE MEDICINE, vol. 7, no. 4, April 2001 (2001-04), pages 471-477, XP002901942
- PIYASENA HEWAWASAM ET AL: "Discovery of a novel class of BK channel openers: Enantiospecific synthesis and BK channel opening activity of 3-(5-chloro-2-hydroxyphenyl)-1,3-dihydro-3 -hydroxy-6-(trifluoromethyl)-2H-indol-2-on e" BIOORGANIC & MEDICINAL CHEMISTRY LETTERS, vol. 7, no. 10, 1997, pages 1255-1260, XP002901943

## Description

### TECHNICAL FIELD

The present invention relates to the use of substituted 3-phenyl oxindole derivatives as modulators of the potassium KCNQ channels.

### BACKGROUND ART

Potassium (K⁺) channels are structurally and functionally diverse families of K⁺-selective channel proteins, which are ubiquitous in cells, indicating their central importance in regulating a number of key cell functions. While widely distributed as a class, K⁺ channels are differentially distributed as individual members of this class or as families.

In general, activation of K⁺ channels in cells leads to hyperpolarization of the cell membrane, or in the case of depolarised cells to repolarization, thereby acting as an endogenous membrane voltage clamp. This is of particular importance in excitable cells such as neurons and muscle cells. K⁺ channels can respond to important cellular events such as changes in the intracellular concentration of ATP or the intracellular concentration of calcium (Ca²⁺). The central role of K⁺ channels in regulating numerous cell functions makes them particularly important targets for therapeutic development.

Thus EP 477819 describes benzimidazole derivatives useful as openers of the BK_{Ca} channel, and EP 747354 discloses 3-substituted oxindole derivatives useful as maxi-K (BK_{Ca}) channel modulators.

Recently another family of potassium channels, the KCNQ channels, has attracted attention as target for therapeutic development. Thus the human KCNQ1 channel has been disclosed by *Wang, Q et al.* [*Wang, Q et al*.; Nature Genet. 1996 **12** 17-23], the human KCNQ2 channel has been disclosed by *Biervert et al.* [*Biervert et al*.; Science 1998 **279** 403-406]; the human KCNQ3 channel has been disclosed by *Schroeder et al.* [*Schroeder et al*.; Nature 1998 **396** 687 -690]; the human KCNQ4 channel has been disclosed by *Kubisch et al.* [*Kubisch et al*.; Cell 1999 **96** (3) 437-46]; and the human KCNQ5 channel has been disclosed by *Schroeder et al.* [*Schroeder et al*.; J. Biol. Chem. on-line 2000 May 17.

Mutated and non-mutated KCNQ2 and KCNQ3 potassium channels have been disclosed in WO 99/07832, WO 99/21875 and WO 99/31232.

Whereas the BK channels belongs to the Slo subfamily of potassium channels, the KCNQ channels constitute a subfamily belonging to the novel KQT family of potassium channels. The different nature of these channels also is established by the fact that compounds acting on BK channels may not necessarily act on the KCNQ channels, and *vice versa*. Thus BK active compounds like the scorpion toxins show no effect on KCNQ channels, and XE-991 and Linopiridine, while being potent KCNQ inhibiting compounds, show no effect on BK channels.

### SUMMARY OF THE INVENTION

According to the present invention it has now been found that a certain group of compounds, formerly known as openers of the BK channels, show excellent KCNQ modulating activities.

It was found, moreover, that these compounds are particular useful for the treatment or alleviation of pain, in particular neuropathic pain, which effect is considered associated with their activity on the KCNQ channels.

In its first aspect the present invention relates to the use of a particular group of 3-substituted oxindole derivatives as modulators of the KCNQ potassium channels, in the manufacture of a medlicament for the treatment, prevention or alleviation of a disease or a disorder or a condition of a living animal body selected from the group consisting of pain, neuropathic pain, chronic headache, central pain, pain related to diabetic neuropathy, to postherpetic neuralgia and to peripheral nerve injury, drug addiction, affective disorders, Alzheimer's disease, anxiety, neurodegenerative illness or diseases, cognitive deficits, compulsive behaviour, dementia, HIV dementia, depression, Huntington's disease, mania, cognitive disorders, memory impairment, memory disorders, memory dysfunction, motion disorders, motor disorders, neurodegenerative diseases, Parkinson's disease, Parkinson-like motor disorders, phobias, Pick's disease, psychosis, a bipolar disorder, schizophrenia, spinal cord damage, cardiomyopathia, cardiac arrhythmia, long QT syndrome, a motion disorder, or a motor disorder, myasthenia gravis, migraine, tension headache, a bowel disorder, an inflammatory disease, ulcerative colitis, Crohn's disease, Creutzfeld-Jacobs disease, an ophthalmic condition, progressive hearing loss or tinnitus, fever, multiple sclerosis, diabetes, or metastatic tumor growth, a pneumoconiosis, such as aluminosis, anthracosisi asbestosis, chalicosis, ptilosis, siderosis, silicosis, tabacosis and byssinosis; and a chronic obstructive pulmonary disease (COPD).

Other objects of the invention will be apparent to the person skilled in the art from the following detailed description and examples.

### DETAILED DISCLOSURE OF THE INVENTION

### KCNQ activating compounds

In the context of this invention a KCNQ activating compound is a substance that is capable of increasing the activity of a KCNQ channel, i.e. the current through the channel, in presence of micromolar or preferably sub-micromolar concentrations of said substance when compared to the activity in absence of said substance, and when determined at physiological conditions.

An increased activity may in particular be determined as an increase of the current obtained under physiologic conditions at any given membrane potential where the channel conducts current under physiological conditions, or as a decreased threshold value for activation of the KCNQ channel.

The activity may be determined by conventional methods for monitoring changes in the membrane potential of a KCNQ channel containing cell, e.g. by electrophysiological methods or by methods based on recording of fluorescence changes.

The KCNQ channel may be a homomeric or heteromeric channel, formed by association of various KCNQ channel subunits, in particular the KCNQ1 subunit, the KCNQ2 subunit, the KCNQ3 subunit, the KCNQ4 subunit and the KCNQ5 subunit.

Preferred homomeric KCNQ channels for use according to the invention are those consisting of KCNQ1 subunits, of KCNQ2 subunits, of KCNQ3 subunits, of KCNQ4 subunits or of KCNQ5 subunits, whereas preferred heteromeric KCNQ channels for use according to the invention are those consisting of KCNQ5 and KCNQ1 channel subunits, of KCNQ5 and KCNQ2 channel subunits, of KCNQ5 and KCNQ3 channel subunits, of KCNQ5 and KCNQ4 channel subunits, of KCNQ5 and KCNQ1 and KCNQ2 channel subunits, of KCNQ5 and KCNQ1 and KCNQ3 channel subunits, of KCNQ5 and KCNQ1 and KCNQ4 channel subunits, of KCNQ5 and KCNQ2 and KCNQ3 channel subunits, of KCNQ5 and KCNQ2 and KCNQ4 channel subunits, of KCNQ5 and KCNQ3 and KCNQ4 channel subunits, of KCNQ5 and KCNQ1 and KCNQ2 and KCNQ3 channel subunits, of KCNQ5 and KCNQ1 and KCNQ2 and KCNQ4 channel subunits, of KCNQ5 and KCNQ1 and KCNQ3 and KCNQ4 channel subunits, or of KCNQ5 and KCNQ2 and KCNQ3 and KCNQ4 channel subunits.

In a preferred embodiment monitoring of the membrane potential of the KCNQ containing cell is performed by patch clamp techniques, e.g. as described by *Hamill, O.P., et al.,* Pflügers Arch. 1981 **351** 85-100.

In other preferred embodiments monitoring of the membrane potential of the KCNQ containing cell is performed using the electrophysiological methods described e.g. in WO 99/19729, in WO 99/31503, in WO 99/34202, in WO 99/64559, in WO 99/66329, in WO 00/34776, in WO 00/71742, or in WO 00/73431.

In another preferred embodiment monitoring of the membrane potential of the KCNQ containing cell is performed using fluorescence methods.

In a preferred embodiment, the KCNQ channel of the KCNQ containing cell is an ion channel that is exogenous to the cell in question, and which cell may in particular be a human embryonic kidney (HEK) cell, a HEK 293 cell, a Chinese hamster ovary (CHO) cell, a *Xenopus laevis* oocyte (XLO) cell, or any other cell line capable of expressing KCNQ channels.

### Oxindole Derivatives

The 3-substituted oxindole derivatives for use according to the invention may be characterised by the general Formula I 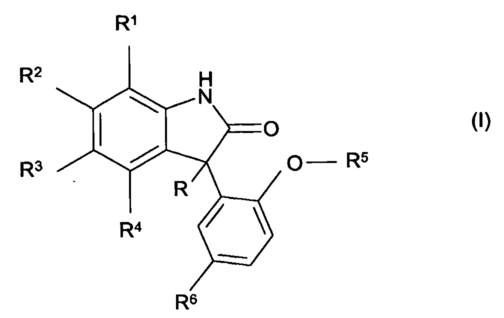 wherein,
R represents hydrogen, halogen or hydroxy,
R¹, R², R³ and R⁴ independently of each another represent hydrogen, halogen alkyl, trihalogenmethyl, phenyl, *p*-methyl-phenyl or *p*-trihalogenmethyl-phenyl, or
R¹ and R², R² and R³, or R³ and R⁴ are joined together to form a benzo fused ring,
R⁵ represents hydrogen or alkyl, and
R⁶ represents halogen or trihalogenmethyl,
or a pharmaceutically-acceptable addition salt thereof.

In a preferred embodiment, the R is hydrogen, fluorine, or a hydroxy group.

In another preferred embodiment, R¹, R², R³ and R⁴ independently of each another represent hydrogen, halogen, alkyl, trihalogenmethyl.

In a third preferred embodiment, R¹, R³ and R⁴ represent hydrogen, and R² represents halogen, trihalogenmethyl or phenyl.

In a fourth preferred embodiment, R¹ and R², R² and R³, or R³ and R⁴ are joined together to form a benzo fused ring.

In a fifth preferred embodiment R⁵ is hydrogen or methyl.

In a fourth preferred embodiment R⁶ is chlorine.

In its most preferred embodiment, the 3-substituted oxindole derivative for use according to the invention is
(+)-3-(5-chloro-2-methoxyphenyl)-1,3-dihydro-6-(trifluoromethyl)-2H-indol-2-one;
(-)-3-(5-chloro-2-methoxyphenyl)-1,3-dihydro-6-(trifluoromethyl)-2H-indol-2-one;
(±)-3-(5-chloro-2-methoxyphenyl)-1,3-dihydro-6-(4-methylphenyl)-2H-indol-2-one;
(±)-3-(5-chloro-2-hydroxyphenyl)-1,3-dihydro-2H-indol-one;
(±)-3-(5-chloro-2-hydroxyphenyl)-4,6-dichloro-1,3-dihydro-2H-indol-one;
(±)-3-(5-chloro-2-hydroxyphenyl)-1,3-dihydro-6-phenyl-2H-indol-one;
(±)-3-(5-chloro-2-hydroxyphenyl)-1,3-dihydro-6-iodo-2H-indol-one;
(±)-3-(5-chloro-2-hydroxyphenyl)-1,3-dihydro-6-(trifluoromethyl)-2H-indol-2-one;
(±)-3-(5-chloro-2-hydroxyphenyl)-1,3-dihydro-6-[4-(trifluoromethyl)-phenyl]-2H-indol-2-one;
(±)-3-(5-chloro-2-hydroxyphenyl)-1,3-dihydro-2H-benz[*e*]indol-2-one;
(±)-3-(5-chloro-2-hydroxyphenyl)-1,3-dihydro-2H-benz[*f*]indol-2-one;
(±)-3-(5-chloro-2-hydroxyphenyl)-1,3-dihydro-2H-benz[*g*]indol-2-one;
(±)-3-(5-chloro-2-methoxyphenyl)-1,3-dihydro-3-hydroxy-6-(trifluoromethyl)-2H-indol-2-one;
(±)-3-(5-chloro-2-methoxyphenyl)-1,3-dihydro-3-hydroxy-4-(trifluoromethyl)-2H-indol-2-one;
(±)-3-(5-chloro-2-methoxyphenyl)-1,3-dihydro-3-hydroxy-6-(trifluoromethyl)-2H-indol-2-one;
(-)-3-(5-chloro-2-methoxyphenyl)-1,3-dihydro-3-hydroxy-6-(trifluoromethyl)-2H-indol-2-one;
(±)-3-(5-chloro-2-methoxyphenyl)-1,3-dihydro-3-hydroxy-7-(trifluoromethyl)-2H-indol-2-one;
(±)-3-(5-chloro-2-methoxyphenyl)-1,3-dihydro-3-hydroxy-5-bromo-2H-indol-2-one;
(±)-3-(5-chloro-2-methoxyphenyl)-1,3-dihydro-3-hydroxy-6-iodo-2H-indol-2-one;
(±)-3-(5-chloro-2-methoxyphenyl)-4,6-dichloro-1,3-dihydro-3-hydroxy-2H-indol-2-one;
(±)-3-(5-chloro-2-methoxyphenyl)-1,3-dihydro-3-hydroxy-2H-benz[*f*]indol-2-one;
(±)-3-(5-chloro-2-hydroxyphenyl)-1,3-dihydro-3-hydroxy-4,6-*bis*-(trifluoromethyl)-2H-indol-2-one;
(±)-3-(5-chloro-2-hydroxyphenyl)-1,3-dihydro-3-hydroxy-4-(trifluoromethyl)-2H-indol-2-one;
(±)-3-(5-chloro-2-hydroxyphenyl)-1,3-dihydro-3-hydroxy-6-(trifluoromethyl)-2H-indol-2-one;
(+)-3-(5-chloro-2-hydroxyphenyl)-1,3-dihydro-3-hydroxy-6-(trifluoromethyl)-2H-indol-2-one;
(-)-3-(5-chloro-2-hydroxyphenyl)-1,3-dihydro-3-hydroxy-6-(trifluoromethyl)-2H-indol-2-one;
(±)-3-(5-chloro-2-hydroxyphenyl)-1,3-dihydro-3-hydroxy-7-(trifluoromethyl)-2H-indol-2-one;
(±)-3-(5-chloro-2-hydroxyphenyl)-4,6-dichloro-1,3-dihydro-3-hydroxy-2H-indol-2-one;
(±)-3-(5-chloro-2-hydroxyphenyl)-1,3-dihydro-3-hydroxy-5-bromo-2H-indol-2-one;
(±)-3-(5-chloro-2-hydroxyphenyl)-1,3-dihydro-3-hydroxy-6-iodo-2H-indol-2-one;
(±)-1,3-dihydro-3-hydroxy-3-[2-hydroxy-5-(trifluoromethyl)-phenyl]-6-(trifluoromethyl)-2H-indol-2-one;
(±)-3-(5-chloro-2-hydroxyphenyl)-1,3-dihydro-3-hydroxy-2H-benz[*g*]indol-2-one;
(±)-3-(5-chloro-2-hydroxyphenyl)-1,3-dihydro-3-hydroxy-2H-benz[*f*]indol-2-one;
(±)-3-(5-chloro-2-methoxyphenyl)-1,3-dihydro-3-fluoro-7-(trifluoromethyl)-2H-indol-2-one;
(±)-3-(5-chloro-2-methoxyphenyl)-1,3-dihydro-3-fluoro-6-phenyl-2H-indol-2-one;
(±)-3-(5-chloro-2-methoxyphenyl)-1,3-dihydro-3-fluoro-6-iodo-2H-indol-2-one;
(±)-3-(5-chloro-2-methoxyphenyl)-1,3-dihydro-3-fluoro-5-methyl-2H-indol-2-one;
(±)-3-(5-chloro-2-methoxyphenyl)-1,3-dihydro-3-fluoro-5-bromo-2H-indol-2-one;
(±)-3-(5-chloro-2-methoxyphenyl)-1,3-dihydro-3-fluoro-4,6-*bis*-(trifluoromethyl)-2H-indol-2-one; or
(±)-3-(5-chloro-2-hydroxyphenyl)-1,3-dihydro-3-fluoro-7-(trifluoromethyl)-2H-indol-2-one.

In its most preferred embodiment, the 3-substituted oxindole derivative for use according to the invention is
(±)-3-(5-chloro-2-methoxyphenyl)-1,3-dihydro-3-fluoro-6-(trifluoromethyl)-2H-indol-2-one (Compound 1); or
(3S)-(+)-(5-chloro-2-methoxyphenyl)-1,3-dihydro-3-fluoro-6-(trifluoromethyl)-2H-indol-2-one (Compound 1A); or
(3R)-(-)-(5-chloro-2-methoxyphenyl)-1,3-dihydro-3-fluoro-6-(trifluoromethyl)-2H-indol-2-one (Compound 1B),
having the following structures:

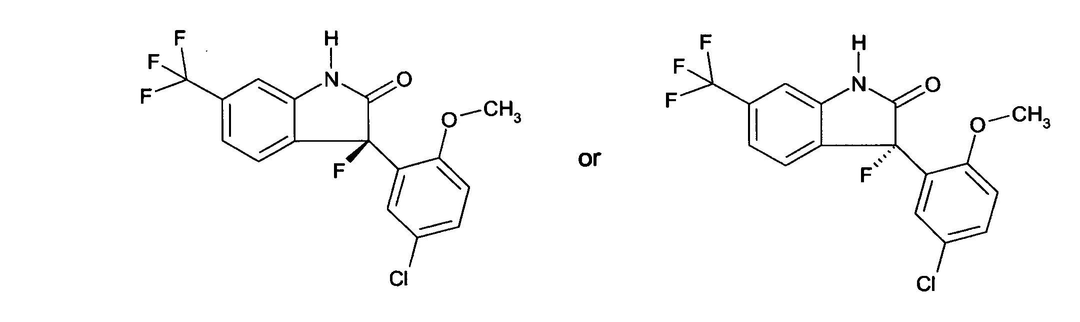

### Definition of Substituents

In the context of this invention halogen represents a fluorine, a chlorine, a bromine or an iodine atom. Thus, a trihalogenmethyl group represents e.g. a trifluoromethyl group and a trichloromethyl group.

In the context of this invention an alkyl group designates a univalent saturated, straight or branched hydrocarbon chain. The hydrocarbon chain preferably contain of from one to eighteen carbon atoms (C₁₋₁₈-alkyl), more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In another preferred embodiment of this invention alkyl represents a C₁₋₃-alkyl group, which may in particular be methyl, ethyl, propyl or isopropyl.

### Pharmaceutically Acceptable Salts

The chemical compound of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride derived from hydrochloric acid, the hydrobromide derived from hydrobromic acid, the nitrate derived from nitric acid, the perchlorate derived from perchloric acid, the phosphate derived from phosphoric acid, the sulphate derived from sulphuric acid, the formate derived from formic acid, the acetate derived from acetic acid, the aconate derived from aconitic acid, the ascorbate derived from ascorbic acid, the benzenesulphonate derived from benzensulphonic acid, the benzoate derived from benzoic acid, the cinnamate derived from cinnamic acid, the citrate derived from citric acid, the embonate derived from embonic acid, the enantate derived from enanthic acid, the fumarate derived from fumaric acid, the glutamate derived from glutamic acid, the glycolate derived from glycolic acid, the lactate derived from lactic acid, the maleate derived from maleic acid, the malonate derived from malonic acid, the mandelate derived from mandelic acid, the methanesulphonate derived from methane sulphonic acid, the naphthalene-2-sulphonate derived from naphtalene-2-sulphonic acid, the phthalate derived from phthalic acid, the salicylate derived from salicylic acid, the sorbate derived from sorbic acid, the stearate derived from stearic acid, the succinate derived from succinic acid, the tartrate derived from tartaric acid, the toluene-p-sulphonate derived from p-toluene sulphonic acid, and the like. Such salts may be formed by procedures well known and described in the art.

Other acids such as oxalic acid, which may not be considered pharmaceutically acceptable, may be useful in the preparation of salts useful as intermediates in obtaining a chemical compound of the invention and its pharmaceutically acceptable acid addition salt.

Metal salts of a chemical compound of the invention includes alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

The chemical compound of the invention may be provided in dissoluble or indissoluble forms together with a pharmaceutically acceptable solvents such as water, ethanol, and the like. Dissoluble forms may also include hydrated forms such as the monohydrate, the dihydrate, the hemihydrate, the trihydrate, the tetrahydrate, and the like. In general, the dissoluble forms are considered equivalent to indissoluble forms for the purposes of this invention.

### Methods of Preparation

The 3-substituted oxindole derivative of the invention may be prepared by conventional methods for chemical synthesis, e.g. those described in EP 747354. The starting materials for the processes described in the present application are known or may readily be prepared by conventional methods from commercially available chemicals.

The end products of the reactions described herein may be isolated by conventional techniques, e.g. by extraction, crystallisation, distillation, chromatography, etc.

### Pharmaceutical Compositions

Viewed from one aspect the invention relates to the use of the 3-substituted oxindole derivative of the general Formula I, or a pharmaceutically-acceptable addition salt thereof, for the manufacture of a pharmaceutical composition for the treatment, prevention or alleviation of a disease or a disorder or a condition of a mammal, including a human, which disease, disorder or condition is responsive to modulation of KCNQ channels.

In a preferred embodiment, the disease, disorder or condition is a disease or adverse condition of the CNS.

In a more preferred embodiment, the disease, disorder or condition include affective disorders, Alzheimer's disease, anxiety, ataxia, neurodegenerative illness or diseases, cognitive deficits, compulsive behaviour, dementia, HIV dementia, depression, Huntington's disease, mania, cognitive disorders, memory impairment, memory disorders, memory dysfunction, motion disorders, motor disorders, neurodegenerative diseases, Parkinson's disease and Parkinson-like motor disorders, phobias, Pick's disease, psychosis, a bipolar disorder, schizophrenia, spinal cord damage, pain, neuropathic pain, central pain, pain related to diabetic neuropathy, to postherpetic neuralgia, or to peripheral nerve injury or drug addiction.

In another preferred embodiment, the disease, disorder or condition is one associated with the heart or skeletal muscle, like cardiomyopathia, cardiac arrhythmia, long QT syndrome, a motion disorder, or a motor disorder, myasthenia gravis, migraine, tension headache, a bowel disorder, an inflammatory disease, ulcerative colitis, Crohn's disease, Creutzfeld-Jacobs disease, an ophthalmic condition, progressive hearing loss or tinnitus, fever, multiple sclerosis, diabetes, metastatic tumor growth, an obstructive or inflammatory airway disease such as an airway hyperreactivity, a pneumoconiosis such as aluminosis, anthracosis, asbestosis, chalicosis, ptilosis, siderosis, silicosis, tabacosis and byssinosis, a chronic obstructive pulmonary disease (COPD).

In yet another preferred embodiment, the 3-substituted oxindole derivative is used for the treatment, prevention or alleviation of migraine, tension headache, progressive hearing loss, tinnitus, or cardiac arrhythmias.

In yet another preferred embodiment, the 3-substituted oxindole derivative is used for the treatment, prevention or alleviation of pain, in particular neuropathic pain, chronic headache, central pain, pain related to diabetic neuropathy, to postherpetic neuralgia, or to peripheral nerve injury.

While the 3-substituted oxindole derivative for use according to the invention may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the 3-substituted oxindole derivative, together with one or more pharmaceutically acceptable carriers therefor, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route which suite the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition may be prepared by the skilled person using standard and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depend on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### Biological Activity

According to the present invention, the 3-substituted oxindole derivatives of Formula I above have been found useful as modulators of the KCNQ potassium channels.

At present five such channels are known, i.e. the KCNQ1 channel, the KCNQ2 channel, the KCNQ3 channel, the KCNQ4 channel, and the KCNQ5 channel, and heteromeric combinations hereof. Moreover, the modulatory activity may be inhibitory (i.e. inhibitory activity) or stimulating (i.e. activating activity).

In a preferred embodiment the 3-substituted oxindole derivatives of the invention show stimulating activity at the KCNQ2, KCNQ3, KCNQ4 and/or the KCNQ5 potassium channels, and the heteromeric combinations hereof.

The modulatory activity may be determined using conventional methods, e.g. binding or activity studies, known in the art, or as described in the working examples.

### BRIEF DESCRIPTION OF THE DRAWINGS

The present invention is further illustrated by reference to the accompanying drawing, in which:
Fig. 1 shows the dose-dependent activation of KCNQ4 stably expressed in HEK-293 cells by Compound 1 (0.1, 0.3, 1, 3 and 10 µM, respectively) [Currents; I/nA vs. time/seconds];
Fig. 2 shows the changes in IV relation and activation curve induced by 3 µM Compound 1;
Fig. 3 shows the hyper-polarisation of a KCNQ4 expressing cell by Compound 1; and
Fig. 4 shows the activation of KCNQ5 stably expressed in HEK-293 cells by Compound 1 [Currents; I/nA vs. time/seconds].

### EXAMPLES

The invention is further illustrated with reference to the following examples which are not intended to be in any way limiting to the scope of the invention as claimed.

### Example 1

### Stable Expression of KCNQ4 Channels and KCNQ5 Channels in HEK-293 Cells

KCNQ4 and KCNQ5 was sub-cloned into the mammalian expression vector pNS1n (NeuroSearch), a custom-designed derivative of pcDNA3Neo (InVitrogen). HEK-293 cells (American Type Culture Collection) were grown in DMEM (Life Technologies) supplemented with 10% FCS (Life Technologies) at 37°C in 5% CO₂.

One day prior to transfection, 10⁶ cells were plated in a cell culture T25 flask (Nunc). Cells were transfected with 2.5 µg of the plasmid pNS1n_KCNQ4 or with 2.5 µg of the plasmid pNS1n_KCNQ5 using Lipofectamine (Life Technologies) according to the manufacturer's instructions. Cells transfected with pNS1n_KCNQ4 or pNS1n_KCNQ5 were selected in media supplemented with 0.5 mg/ml geneticin (G418; Life Technologies).

Single clones were picked and propagated in selection media for five passages after which they were considered stable. Following the cells were cultured in regular medium without selection agent.

Expression of functional KCNQ4 channels or KCNQ5 channels was verified by patch-clamp measurements.

### Example 2

### Electrophysiology

This example demonstrates the electrophysiological activity of a KCNQ4 or a KCNQ5 containing cell, obtained as described in Example 1, when subjected to a compound representative of the invention, (±)-3-(5-chloro-2-methoxyphenyl)-1,3-dihydro-3-fluoro-6-(trifluoromethyl)-2H-indol-2-one (Compound 1), obtained according to Example 14 of EP 747,354.

Transfected cells were cultured on glass cover slips (Ø=3mm), which were transferred to a small recording chamber. The chamber was perfused with extracellular solutions at a rate of 1 ml/min giving rise to full exchange of the chamber volume (15 µl) each second.

Whole-cell currents were recorded using using a HEKA EPC-9 amplifier and borosilicate glass micropipettes with tip resistances of 1.5-3 MΩ. Series resistances were below 5 MΩ, mostly stayed constant during the experiment and were compensated by 70%. The currents were not leak-subtracted.

All experiments were performed at room temperature (21-25°C). Igor software (WaveMetrics, Lake Oswego, OR) was used for the analysis.

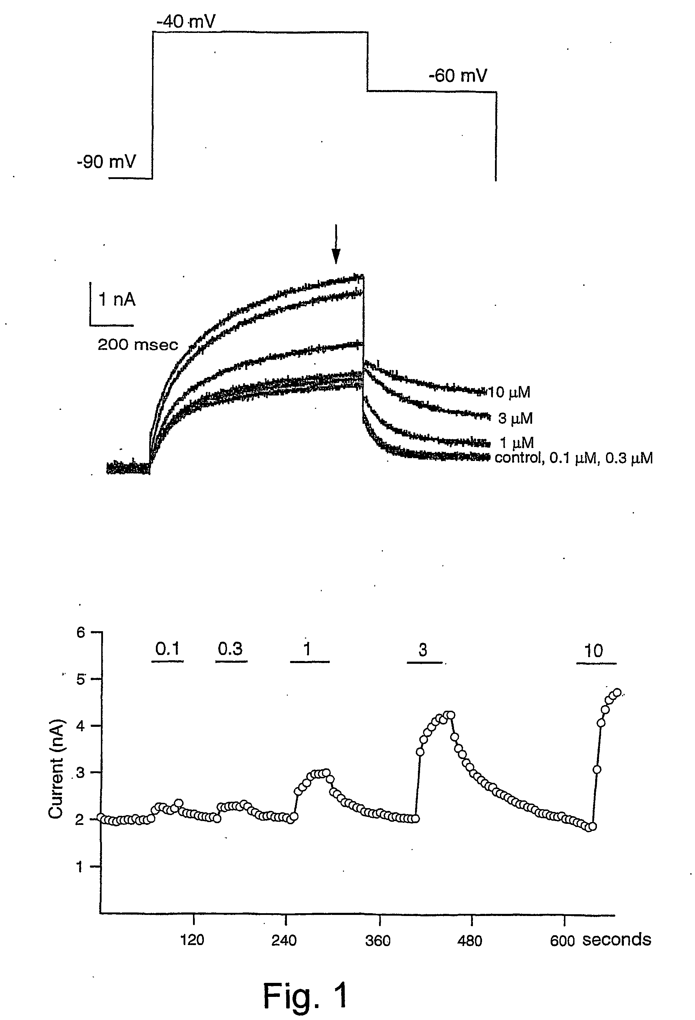

Compound 1 was present in the bath solution during for a period as indicated by the bars, and in the concentrations (µM) listed in Figs. 1-4.

The bath solution consisted of 144 mM NaCl, 4 mM KCI, 2 mM CaCl₂, 1 mM MgCl₂ and 10 mM HEPES (pH adjusted to 7.4). The pipette solution consisted of 144 mM KCI, 5.4 mM CaCl₂, 1.8 mM MgCl₂, 4 mM NaATP, 0.4 mM GTP, 10 mM EGTA and 10 mM HEPES (pH adjusted to 7.2).

### Dose-dependent activation of KCNQ4 (cf. Fig. 1)

The currents were measured in voltage mode using the whole-cell configuration of the patch clamp technique. The holding potential was -90 mV and the currents were elicited by a 1 second depolarising step to -40 mV followed by a 500 msecond step to -60 mV repeated every 5 seconds (upper panel). The panel in the middle shows current traces recorded in the absence (control) and in the presence of the indicated concentrations of Compound 1. The lower panel shows the time course of the experiment with the currents recorded at the end of the step to -40 mV (indicated by the arrow in the middle panel) as a function of time.

The result of this experiment is presented in Fig. 1.

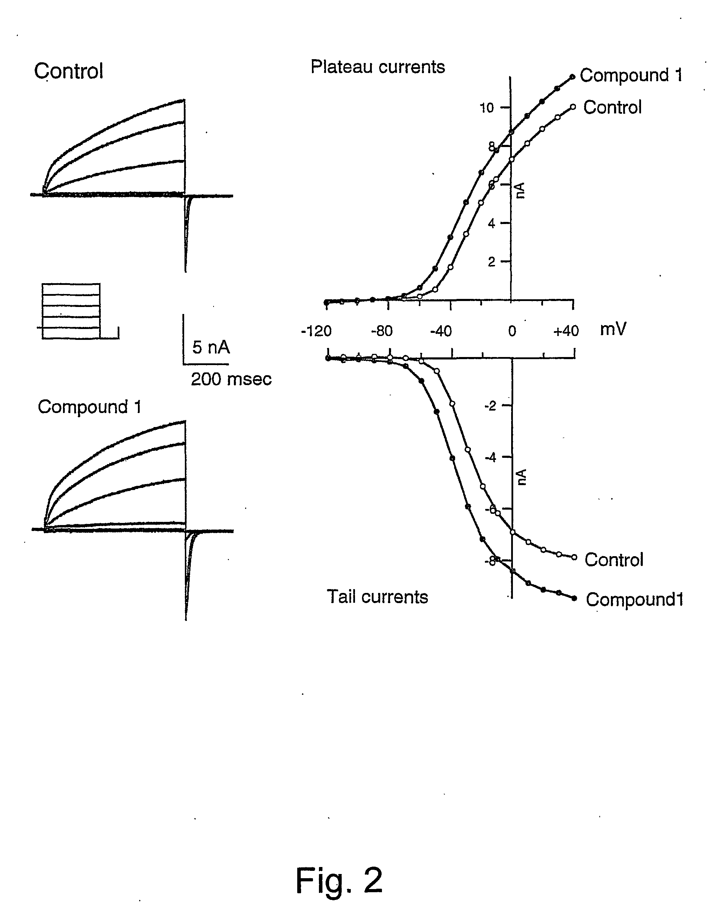

### Changes in IV Relation and Activation Curve (Fig. 2)

The experimental conditions was as described above, but in this experiment the depolarising potential ranged from -120 mV to +40 mV before a step back to -120 mV where brief inward tail currents were recorded.

The current traces in the absence (control) and in the presence of 3 µM Compound 1 are shown for the steps to -120, -90, -60, -30, 0, and +30 mV. The upper right panel shows the size of the currents measured at the plateau as a function of the depolarising potential.

The IV-relation in the absence of compound is shown as open circles, whereas the IV curve in the presence of 3 µM Compound 1 is shown as closed circles. The lower right panel shows the peak size of the tail currents as a function of the preceding activating potential (Open circles: Control, Closed circles: 3 µM Compound 1).

The result of this experiment is presented in Fig. 2.

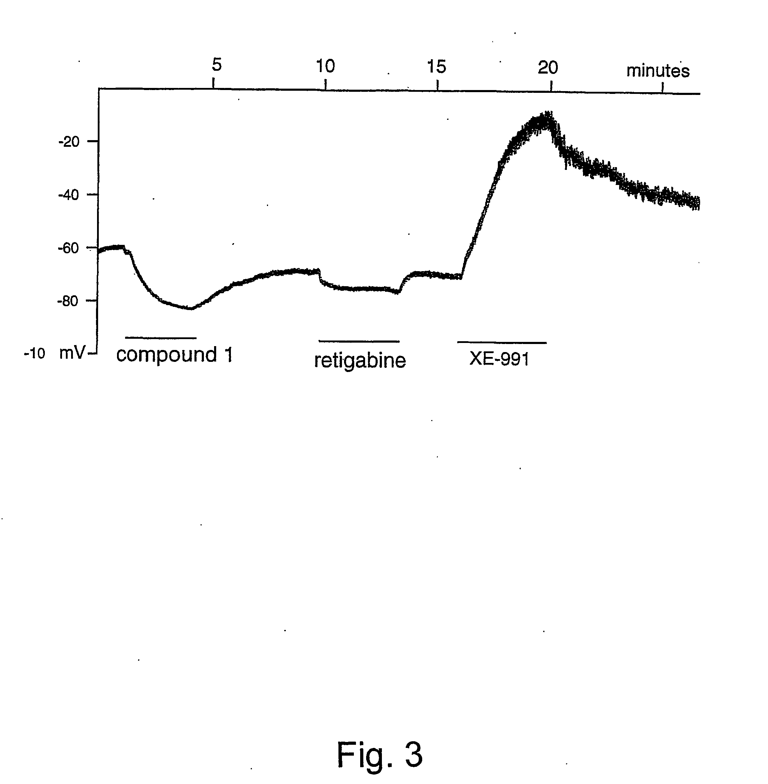

### Hyperpolarisation of a KCNQ4 Expressing Cell (Fig. 3)

The membrane potential was measured in current clamp mode using the whole cell configuration of the patch clamp technique. A resting membrane potential of around -60 mV was initially recorded from this cell and the addition of 10 µM Compound 1 to the bath solution induced a hyper-polarisation of the membrane potential to -83 mV.

After washout of Compound 1, the membrane potential returned to -69 mV, and subsequent addition of 10 µM Retigabine, a KCNQ activator, induced a faster but smaller hyperpolarisation to a membrane potential of -76 mV.

After washout of Retigabine, 10 µM of XE-991, a potent KCNQ inhibiting compound, was added and this induced a depolarisation to -12 mV, which membrane potential returned very slowly towards the control level upon washout.

The compounds were present in the bath solution during the times indicated by the bars.

The result of this experiment is presented in Fig. 3.

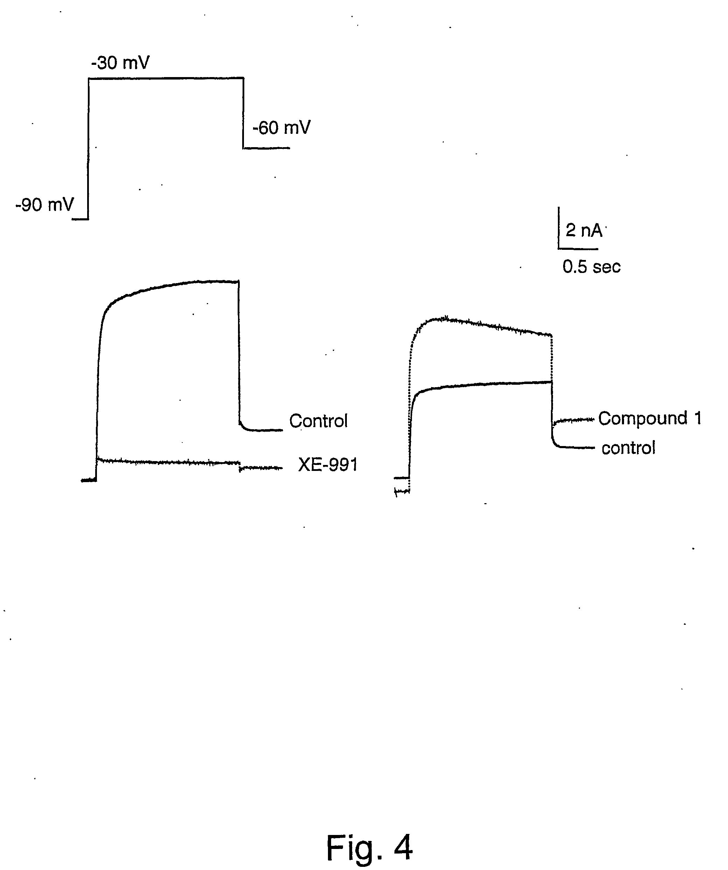

### Activation of KCNQ5 in HEK-293 Cells (Fig. 4)

The currents were measured from a cell patch clamped in the whole-cell voltage clamp configuration. The holding potential was -90 mV and the currents were activated by a 2 second depolarising step to -30 mV followed by a 500 msecond step to -60 mV (upper panel).

The currents recorded in the absence (control) and in the presence of 10 µM of the reference M current blocker XE-991 is shown in the panel to the left. The panel to the right shows the activation by 10 µM Compound 1 to the same cell after washout of XE-991. The inhibition by XE-991 was not fully reversible and the control current measured immediately before addition of Compound 1 was therefore smaller than the initial control current.

The result of this experiment is presented in Fig. 4.

## Claims

1. Use of a 3-substituted oxindole derivative having the general Formula I, 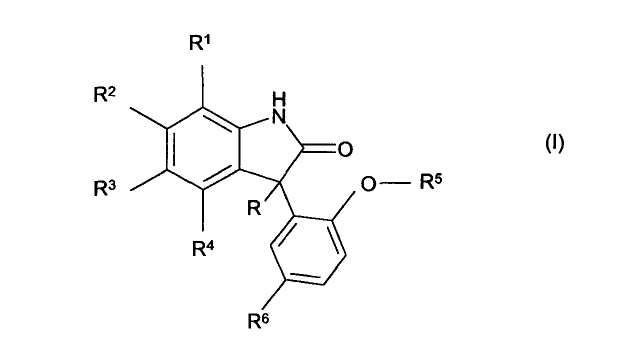 wherein,
R represents hydrogen, halogen or hydroxy,
R¹, R², R³ and R⁴ independently of each another represent hydrogen, halogen, alkyl, trihalogenmethyl, phenyl, *p*-methyl-phenyl or *p*-trihalogenmethyl-phenyl, or
R¹ and R², R² and R³, or R³ and R⁴ are joined together to form a benzo fused ring,
R⁵ represents hydrogen or alkyl, and
R⁶ represents halogen or trihalogenmethyl,
or a pharmaceutically-acceptable addition salt thereof,
wherein said oxindole derivative is capable of modulating the ion flow through KCNQ channels,
for the manufacture of a pharmaceutical composition,
for the treatment, prevention or alleviation of a disease or a disorder or a condition of a living animal body selected from the group consisting of pain, neuropathic pain, chronic headache, central pain, pain related to diabetic neuropathy, to postherpetic neuralgia and to peripheral nerve injury, drug addiction, affective disorders, Alzheimer's disease, anxiety, neurodegenerative illness or diseases, cognitive deficits, compulsive behaviour, dementia, HIV dementia, depression, Huntington's disease, mania, cognitive disorders, memory impairment, memory disorders, memory dysfunction, motion disorders, motor disorders, neurodegenerative diseases, Parkinson's disease, Parkinson-like motor disorders, phobias, Pick's disease, psychosis, a bipolar disorder, schizophrenia, spinal cord damage, cardiomyopathia, cardiac arrhythmia, long QT syndrome, a motion disorder, or a motor disorder, myasthenia gravis, migraine, tension headache, a bowel disorder, an inflammatory disease, ulcerative colitis, Crohn's disease, Creutzfeld-Jacobs disease, an ophthalmic condition, progressive hearing loss or tinnitus, fever, multiple sclerosis, diabetes, or metastatic tumor growth, a pneumoconiosis, such as aluminosis, anthracosis, asbestosis, chalicosis, ptilosis, siderosis, silicosis, tabacosis and byssinosis; and a chronic obstructive pulmonary disease (COPD).

2. Use according to claim 1, wherein the said disease, disorder or condition is selected from the group consisting of pain, neuropathic pain, chronic headache, central pain, pain related to diabetic neuropathy, to postherpetic neuralgia and to peripheral nerve injury.

3. Use according to claim 1, wherein
R represents hydrogen, halogen or hydroxy,
R¹, R², R³ and R⁴ independently of each another represent hydrogen, halogen, alkyl, trihalogenmethyl, phenyl, *p*-methyl-phenyl or *p*-trihalogenmethyl-phenyl,
and when R¹ and R⁴ are hydrogen, then R² or R³ is phenyl, *p*-methyl-phenyl or *p*-trihalogenmethyl-phenyl; or
R¹ and R², R² and R³, or R³ and R⁴ are joined together to form a benzo fused ring,
R⁵ represents hydrogen or alkyl, and
R⁶ represents halogen or trihalogenmethyl.

4. Use according to claim 1, wherein
R¹, R³ and R⁴ represent hydrogen, and
R² represents halogen, trihalogenmethyl or phenyl.

5. Use according to claim 1, wherein
R¹ and R², R² and R³, or R³ and R⁴ are joined together to form a benzo fused ring.

6. Use according to any of claims 1-4, wherein
R⁵ is hydrogen or methyl.

7. Use according to any of claims 1-5, wherein
R⁶ is chlorine.

8. Use according to claim 1, wherein 3-substituted oxindole derivative for use according to the invention is
(+)-3-(5-chloro-2-methoxyphenyl)-1,3-dihydro-6-(trifluoromethyl)-2H-indol-2-one;
(-)-3-(5-chloro-2-methoxyphenyl)-1,3-dihydro-6-(trifluoromethyl)-2H-indol-2-one;
(±)-3-(5-chloro-2-methoxyphenyl)-1,3-dihydro-6-(4-methylphenyl)-2H-indol-2-one;
(±)-3-(5-chloro-2-hydroxyphenyl)-1,3-dihydro-2H-indol-one;
(±)-3-(5-chloro-2-hydroxyphenyl)-4,6-dichloro-1,3-dihydro-2H-indol-one;
(±)-3-(5-chloro-2-hydroxyphenyl)-1,3-dihydro-6-phenyl-2H-indol-one;
(±)-3-(5-chloro-2-hydroxyphenyl)-1,3-dihydro-6-iodo-2H-indol-one;
(±)-3-(5-chloro-2-hydroxyphenyl)-1,3-dihydro-6-(trifluoromethyl)-2H-indol-2-one;
(±)-3-(5-chloro-2-hydroxyphenyl)-1,3-dihydro-6-[4-(trifluoromethyl)-phenyl]-2H-indol-2-one;
(±)-3-(5-chloro-2-hydroxyphenyl)-1,3-dihydro-2H-benz[*e*]indol-2-one;
(±)-3-(5-chloro-2-hydroxyphenyl)-1,3-dihydro-2H-benz[*f*]indol-2-one;
(±)-3-(5-chloro-2-hydroxyphenyl)-1,3-dihydro-2H-benz[*g*]indol-2-one;
(±)-3-(5-chtoro-2-methoxyphenyl)-1,3-dihydro-3-hydroxy-6-(trifluoromethyl)-2H-indol-2-one;
(±)-3-(5-chloro-2-methoxyphenyl)-1,3-dihydro-3-hydroxy-4-(trifluoromethyl)-2H-indol-2-one;
(±)-3-(5-chloro-2-methoxyphenyl)-1,3-dihydro-3-hydroxy-6-(trifluoromethyl)-2H-indol-2-one;
(-)-3-(5-chloro-2-methoxyphenyl)-1,3-dihydro-3-hydroxy-6-(trifluoromethyl)-2H-indol-2-one;
(±)-3-(5-chloro-2-methoxyphenyl)-1,3-dihydro-3-hydroxy-7-(trifluoromethyl)-2H-indol-2-one;
(±)-3-(5-chloro-2-methoxyphenyl)-1,3-dihydro-3-hydroxy-5-bromo-2H-indol-2-one;
(±)-3-(5-chloro-2-methoxyphenyl)-1,3-dihydro-3-hydroxy-6-iodo-2H-indol-2-one;
(±)-3-(5-chloro-2-methoxyphenyl)-4,6-dichloro-1,3-dihydro-3-hydroxy-2H-indol-2-one;
(±)-3-(5-chloro-2-methoxyphenyl)-1,3-dihydro-3-hydroxy-2H-benz[*f*]indol-2-one;
(±)-3-(5-chloro-2-hydroxyphenyl)-1,3-dihydro-3-hydroxy-4,6-*bis*-(trifluoromethyl)-2H-indol-2-one;
(±)-3-(5-chloro-2-hydroxyphenyl)-1,3-dihydro-3-hydroxy-4-(trifluoromethyl)-2H-indol-2-one;
(±)-3-(5-chloro-2-hydroxyphenyl)-1,3-dihydro-3-hydroxy-6-(trifluoromethyl)-2H-indol-2-one;
(+)-3-(5-chloro-2-hydroxyphenyl)-1,3-dihydro-3-hydroxy-6-(trifluoromethyl)-2H-indol-2-one;
(-)-3-(5-chloro-2-hydroxyphenyl)-1,3-dihydro-3-hydroxy-6-(trifluoromethyl)-2H-indol-2-one;
(±)-3-(5-chloro-2-hydroxyphenyl)-1,3-dihydro-3-hydroxy-7-(trifluoromethyl)-2H-indol-2-one;
(±)-3-(5-chloro-2-hydroxyphenyl)-4,6-dichloro-1,3-dihydro-3-hydroxy-2H-indol-2-one;
(±)-3-(5-chloro-2-hydroxyphenyl)-1,3-dihydro-3-hydroxy-5-bromo-2H-indol-2-one;
(±)-3-(5-chloro-2-hydroxyphenyl)-1,3-dihydro-3-hydroxy-6-iodo-2H-indol-2-one;
(±)-1,3-dihydro-3-hydroxy-3-[2-hydroxy-5-(trifluoromethyl)-phenyl]-6-(trifluoromethyl)-2H-indol-2-one;
(±)-3-(5-chloro-2-hydroxyphenyl)-1,3-dihydro-3-hydroxy-2H-benz[*g*]indol-2-one;
(±)-3-(5-chloro-2-hydroxyphenyl)-1,3-dihydro-3-hydroxy-2H-benz[*f*]indol-2-one;
(±)-3-(5-chloro-2-methoxyphenyl)-1,3-dihydro-3-fluoro-6-(trifluoromethyl)-2H-indol-2-one;
(3S)-(+)-(5-chloro-2-methoxyphenyl)-1,3-dihydro-3-fluoro-6-(trifluoromethyl)-2H-indol-2-one;
(3R)-(-)-(5-chloro-2-methoxyphenyl)-1,3-dihydro-3-fluoro-6-(trifluoromethyl)-2H-indol-2-one;
(±)-3-(5-chloro-2-methoxyphenyl)-1,3-dihydro-3-fluoro-7-(trifluoromethyl)-2H-indol-2-one;
(±)-3-(5-chloro-2-methoxyphenyl)-1,3-dihydro-3-fluoro-6-phenyl-2H-indol-2-one;
(±)-3-(5-chloro-2-methoxyphenyl)-1,3-dihydro-3-fluoro-6-iodo-2H-indol-2-one;
(±)-3-(5-chloro-2-methoxyphenyl)-1,3-dihydro-3-fluoro-5-methyl-2H-indol-2-one;
(±)-3-(5-chloro-2-methoxyphenyl)-1,3-dihydro-3-fluoro-5-bromo-2H-indol-2-one;
(±)-3-(5-chloro-2-methoxyphenyl)-1,3-dihydro-3-fluoro-4,6-*bis*-(trifluoromethyl)-2H-indol-2-one; or
(±)-3-(5-chloro-2-hydroxyphenyl)-1,3-dihydro-3-fluoro-7-(trifluoromethyl)-2H-indol-2-one;
or a pharmaceutically-acceptable addition salt thereof.

9. Use according to claim 1, wherein
R represents hydrogen, halogen or hydroxy;
R¹, R³ and R⁴ represent hydrogen;
R² represents halogen, trihalogenmethyl or phenyl;
R⁵ represents hydrogen or methyl; and
R⁶ represents chlorine.

10. Use according to claim 9, wherein 3-substituted oxindole derivative is
(+)-3-(5-chloro-2-methoxyphenyl)-1,3-dihydro-6-(trifluoromethyl)-2H-indol-2-one;
(-)-3-(5-chloro-2-methoxyphenyl)-1,3-dihydro-6-(trifluoromethyl)-2H-indol-2-one;
(±)-3-(5-chloro-2-hydroxyphenyl)-1,3-dihydro-6-phenyl-2H-indol-one;
(±)-3-(5-chloro-2-hydroxyphenyl)-1,3-dihydro-6-iodo-2H-indol-one;
(±)-3-(5-chloro-2-hydroxyphenyl)-1,3-dihydro-6-(trifluoromethyl)-2H-indol-2-one;
(±)-3-(5-chloro-2-methoxyphenyl)-1,3-dihydro-3-hydroxy-6-(trifluoromethyl)-2H-indol-2-one;
(±)-3-(5-chloro-2-methoxyphenyl)-1,3-dihydro-3-hydroxy-6-(trifluoromethyl)-2H-indol-2-one;
(-)-3-(5-chloro-2-methoxyphenyl)-1,3-dihydro-3-hydroxy-6-(trifluoromethyl)-2H-indol-2-one;
(±)-3-(5-chloro-2-methoxyphenyl)-1,3-dihydro-3-hydroxy-6-iodo-2H-indol-2-one;
(±)-3-(5-chloro-2-hydroxyphenyl)-1,3-dihydro-3-hydroxy-6-(trifluoromethyl)-2H-indol-2-one;
(+)-3-(5-chloro-2-hydroxyphenyl)-1,3-dihydro-3-hydroxy-6-(trifluoromethyl)-2H-indol-2-one;
(-)-3-(5-chloro-2-hydroxyphenyl)-1,3-dihydro-3-hydroxy-6-(trifluoromethyl)-2H-indol-2-one;
(±)-3-(5-chloro-2-hydroxyphenyl)-1,3-dihydro-3-hydroxy-6-iodo-2H-indol-2-one;
(±)-3-(5-chloro-2-methoxyphenyl)-1,3-dihydro-3-fluoro-6-(trifluoromethyl)-2H-indol-2-one;
(3S)-(+)-(5-chloro-2-methoxyphenyl)-1,3-dihydro-3-fluoro-6-(trifluoromethyl)-2H-indol-2-one;
(3R)-(-)-(5-chloro-2-methoxyphenyl)-1,3-dihydro-3-fluoro-6-(trifluoromethyl)-2H-indol-2-one;
(±)-3-(5-chloro-2-methoxyphenyl)-1,3-dihydro-3-fluoro-6-phenyl-2H-indol-2-one; or
(±)-3-(5-chloro-2-methoxyphenyl)-1,3-dihydro-3-fluoro-6-iodo-2H-indol-2-one;
or a pharmaceutically-acceptable addition salt thereof.

11. Use according to claim 9, wherein 3-substituted oxindole derivative is
(±)-3-(5-chloro-2-methoxyphenyl)-1,3-dihydro-3-fluoro-6-(trifluoromethyl)-2H-indol-2-one;
(3S)-(+)-(5-chloro-2-methoxyphenyl)-1,3-dihydro-3-fluoro-6-(trifluoromethyl)-2H-indol-2-one; or
(3R)-(-)-(5-chloro-2-methoxyphenyl)-1,3-dihydro-3-fluoro-6-(trifluoromethyl)-2H-indol-2-one;
or a pharmaceutically-acceptable addition salt thereof.

## Patentansprüche

1. Verwendung eines 3-substituierten Oxindol-Derivats mit der allgemeinen Formel I, 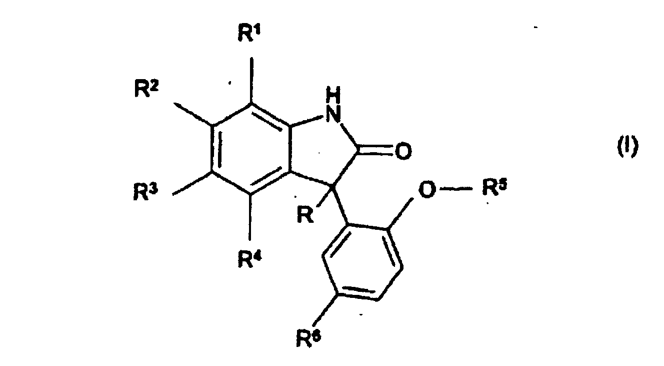 worin,
R Wasserstoff, Halogen oder Hydroxy darstellt,
R¹, R², R³ und R⁴ unabhängig voneinander Wasserstoff, Halogen, Alkyl, Trihalogenmethyl, Phenyl, *p*-Methyl-phenyl oder *p*-Trihalogenmethyl-phenyl darstellen,
oder
R¹ und R², R² und R³ oder R³ und R⁴ miteinander verbunden sind, um einen benzokondensierten Ring zu bilden,
R⁵ Wasserstoff oder Alkyl darstellt, und
R⁶ Halogen oder Trihalogenmethyl darstellt,
oder ein pharmazeutisch akzeptables Additionssalz davon,
worin das Oxindol-Derivat fähig ist, den Ionenfluss durch KCNQ-Kanäle zu modulieren,
für die Herstellung einer pharmazeutischen Zusammensetzung,
für die Behandlung, Vorbeugung oder Linderung einer Krankheit oder einer Störung oder eines Zustands bzw. eines Leidens eines lebenden tierischen Körpers ausgewählt aus der Gruppe bestehend aus Schmerz, neuropathischem Schmerz, chronischem Kopfschmerz, zentralem Schmerz, Schmerz in Verbindung mit diabetischer Neuropathie, mit postherpetischer Neuralgie und mit peripherer Nervenverletzung, Drogenabhängigkeit, affektiven Störungen, Alzheimer-Krankheit, Angstzustand, neurodegenerativer Erkrankung oder Krankheiten, kognitive Defizite, zwanghaftes Verhalten, Demenz, HIV-Demenz, Depression, Huntington-Krankheit, Manie, kognitive Störungen, Gedächtnisschwäche, Gedächtnisstörungen, Gedächtnisfehlfunktion, Bewegungsstörungen, motorischen Störungen, neurodegenerativen Krankheiten, Parkinson-Krankheit, Parkinson-ähnlichen motorischen Störungen, Phobien, Pick'scher Krankheit, Psychose, einer bipolaren Störung, Schizophrenie, Rückenmarksschaden, Kardiomyopathie, Herzrhythmusstörung (Herzarrhythmie), langem QT-Syndrom, einer Bewegungsstörung, oder einer motorischen Störung, Myasthenia gravis pseudoparalytica, Migräne, Spannungskopfschmerz, einer Darmstörung, einer entzündlichen Krankheit, Colitis ulcerosa, Morbus Crohn, Creutzfeldt-Jacob-Krankheit, einem Augenleiden, fortschreitendem Hörverlust oder Ohrensausen (Tinnitus), Fieber, Multipler Sklerose, Diabetes oder Tumormetastasenwachstum, einer Pneumokoniose, wie beispielsweise Aluminose, Anthrakose, Asbestose, Chalikose, Ptilose, Siderose, Silikose, Tabakose (Tabakvergiftung) und Byssinose, und einer chronisch obstruktiven Lungenkrankheit (COPD).

2. Verwendung gemäß Anspruch 1, worin die Krankheit, die Störung oder der Zustand ausgewählt ist aus der Gruppe bestehend aus Schmerz, neuropathischem Schmerz, chronischem Kopfschmerz, zentralem Schmerz, Schmerz in Verbindung mit diabetischer Neuropathie, mit postherpetischer Neuralgie und mit peripherer Nervenverletzung.

3. Verwendung gemäß Anspruch 1, worin
R Wasserstoff, Halogen oder Hydroxy darstellt,
R¹, R², R³ und R⁴ unabhängig voneinander Wasserstoff, Halogen, Alkyl, Trihalogenmethyl, Phenyl, *p*-Methyl-phenyl oder *p*-Trihalogenmethyl-phenyl darstellen,
und R² oder R³ Phenyl, *p*-Methyl-phenyl oder *p*-Trihalogenmethyl-phenyl sind, wenn R¹ und R⁴ Wasserstoff darstellen, oder
R¹ und R², R² und R³ oder R³ und R⁴ miteinander verbunden sind, um einen benzokondensierten Ring zu bilden,
R⁵ Wasserstoff oder Alkyl darstellt, und
R⁶ Halogen oder Trihalogenmethyl darstellt.

4. Verwendung gemäß Anspruch 1, worin
R¹, R³ und R⁴ Wasserstoff darstellen, und
R² Halogen, Trihalogenmethyl oder Phenyl darstellt.

5. Verwendung gemäß Anspruch 1, worin
R¹ und R², R² und R³ oder R³ und R⁴ miteinander verbunden sind, um einen benzokondensierten Ring zu bilden.

6. Verwendung gemäß einem der Ansprüche 1-4, worin
R⁵ Wasserstoff oder Methyl darstellt.

7. Verwendung gemäß einem der Ansprüche 1-5, worin
R⁶ Chlor darstellt.

8. Verwendung gemäß Anspruch 1, worin das 3-substitutierte Oxindol-Derivat zur Verwendung gemäß der Erfindung
(+)-3-(5-Chlor-2-methoxyphenyl)-1,3-dihydro-6-(trifluormethyl)-2H-indol-2-on,
(-)-3-(5-Chlor-2-methoxyphenyl)-1,3-dihydro-6-(trifluormethyl)-2H-indol-2-on,
(±)-3-(5-Chlor-2-methoxyphenyl)-1,3-dihydro-6-(4-methylphenyl)-2H-indol-2-on,
(±)-3-(5-Chlor-2-hydroxyphenyl)-1,3-dihydro-2H-indol-on,
(±)-3-(5-Chlor-2-hydroxyphenyl)-4,6-dichloro-1,3-dihydro-2H-indol-on,
(±)-3-(5-Chlor-2-hydroxyphenyl)-1,3-dihydro-6-phenyl-2H-indol-on,
(±)-3-(5-Chlor-2-hydroxyphenyl)-1,3-dihydro-6-iod-2H-indol-on,
(±)-3-(5-Chlor-2-hydroxyphenyl)-1,3-dihydro-6-(trifluonnethyl)-2H-indol-2-on,
(±)-3-(5-Chlor-2-hydroxyphenyl)-1,3-dihydro-6-[4-(trifluormethyl)-phenyl]-2H-indol-2-on,
(±)-3-(5-Chlor-2-hydroxyphenyl)-1,3-dihydro-2H-benz[*e*]indol-2-on,
(±)-3-(5-Chlor-2-hydroxyphenyl)-1,3-dihydro-2H-benz[*f*]indol-2-on,
(±)-3-(5-Chlor-2-hydroxyphenyl)-1,3-dihydro-2H-benz[*g*]indol-2-on,
(±)-3-(5-Chlor-2-methoxyphenyl)-1,3-dihydro-3-hydroxy-6-(trifluormethyl)-2H-indol-2-on,
(±)-3-(5-Chlor-2-methoxyphenyl)-1,3-dihydro-3-hydroxy-4-(trifluormethyl)-2H-indol-2-on,
(±)-3-(5-Chlor-2-methoxyphenyl)-1,3-dihydro-3-hydroxy-6-(trifluormethyl)-2H-indol-2-on,
(-)-3-(5-Chlor-2-methoxyphenyl)-1,3-dihydro-3-hydroxy-6-(trifluormethyl)-2H-indol-2-on,
(±)-3-(5-Chlor-2-methoxyphenyl)-1,3-dihydro-3-hydroxy-7-(trifluormethyl)-2H-indol-2-on,
(±)-3-(5-Chlor-2-methoxyphenyl)-1,3-dihydro-3-hydroxy-5-brom-2H-indol-2-on,
(±)-3-(5-Chlor-2-methoxyphenyl)-1,3-dihydro-3-hydroxy-6-iod-2H-indol-2-on,
(±)-3-(5-Chlor-2-methoxyphenyl)-4,6-dichlor-1,3-dihydro-3-hydroxy-2H-indol-2-on,
(±)-3-(5-Chlor-2-methoxyphenyl)-1,3-dihydro-3-hydroxy-2H-benz[*f*]indol-2-on,
(±)-3-(5-Chlor-2-hydroxyphenyl)-1,3-dihydro-3-hydroxy-4,6-*bis*-(trifluormethyl)-2H-indol-2-on,
(±)-3-(5-Chlor-2-hydroxyphenyl)-1,3-dihydro-3-hydroxy-4-(trifluormethyl)-2H-indol-2-on,
(±)-3-(5-Chlor-2-hydroxyphenyl)-1,3-dihydro-3-hydroxy-6-(trifluormethyl)-2H-indol-2-on,
(+)-3-(5-Chlor-2-hydroxyphenyl)-1,3-dihydro-3-hydroxy-6-(trifluormethyl)-2H-indol-2-on,
(-)-3-(5-Chlor-2-hydroxyphenyl)-1,3-dihydro-3-hydroxy-6-(trifluormethyl)-2H-indol-2-on,
(±)-3-(5-Chlor-2-hydroxyphenyl)-1,3-dihydro-3-hydroxy-7-(trifluormethyl)-2H-indol-2-on,
(±)-3-(5-Chlor-2-hydroxyphenyl)-4,6-dichlor-1,3-dihydro-3-hydroxy-2H-indol-2-on,
(±)-3-(5-Chlor-2-hydroxyphenyl)-1,3-dihydro-3-hydroxy-5-brom-2H-indol-2-on,
(±)-3-(5-Chlor-2-hydroxyphenyl)-1,3-dihydro-3-hydroxy-6-iod-2H-indol-2-on,
(±)-1,3-Dihydro-3-hydroxy-3-[2-hydroxy-5-(trifluormethyl)-phenyl]-6-(trifluormethyl)-2H-indol-2-on,
(±)-3-(5-Chlor-2-hydroxyphenyl)-1,3-dihydro-3-hydroxy-2H-benz[*g*]indol-2-on,
(±)-3-(5-Chlor-2-hydroxyphenyl)-1,3-dihydro-3-hydroxy-2H-benz[*f*]indol-2-on,
(±)-3-(5-Chlor-2-methoxyphenyl)-1,3-dihydro-3-fluor-6-(trifluormethyl)-2H-indol-2-on,
(3S)-(+)-(5-Chlor-2-methoxyphenyl)-1,3-dihydro-3-fluor-6-(trifluormethyl)-2H-indol-2-on,
(3R)-(-)-(5-Chlor-2-methoxyphenyl)-1,3-dihydro-3-fluor-6-(trifluormethyl)-2H-indol-2-on,
(±)-3-(5-Chlor-2-methoxyphenyl)-1,3-dihydro-3-fluor-7-(trifluormethyl)-2H-indol-2-on,
(±)-3-(5-Chlor-2-methoxyphenyl)-1,3-dihydro-3-fluor-6-phenyl-2H-indol-2-on,
(±)-3-(5-Chlor-2-methoxyphenyl)-1,3-dihydro-3-fluor-6-iod-2H-indol-2-on,
(±)-3-(5-Chlor-2-methoxyphenyl)-1,3-dihydro-3-fluor-5-methyl-2H-indol-2-on,
(±)-3-(5-Chlor-2-methoxyphenyl)-1,3-dihydro-3-fluor-5-brom-2H-indol-2-on,
(±)-3-(5-Chlor-2-methoxyphenyl)-1,3-dihydro-3-fluor-4,6-bis-(trifluormethyl)-2H-indol-2-on, oder
(±)-3-(5-Chlor-2-hydroxyphenyl)-1,3-dihydro-3-fluor-7-(trifluormethyl)-2H-indol-2-on,
oder ein pharmazeutisch-akzeptables Additionssalz davon ist.

9. Verwendung gemäß Anspruch 1, worin
R Wasserstoff, Halogen oder Hydroxy darstellt,
R¹, R³ and R⁴ Wasserstoff darstellen,
R² Halogen, Trihalogenmethyl oder Phenyl darstellt,
R⁵ Wasserstoff oder Methyl darstellt, und
R⁶ Chlor darstellt.

10. Verwendung gemäß Anspruch 9, worin das 3-substituierte Oxindol-Derivat
(+)-3-(5-Chlor-2-methoxyphenyl)-1,3-dihydro-6-(trifluormethyl)-2H-indol-2-on,
(-)-3-(5-Chlor-2-methoxyphenyl)-1,3-dihydro-6-(trifluormethyl)-2H-indol-2-on,
(±)-3-(5-Chlor-2-hydroxyphenyl)-1,3-dihydro-6-phenyl-2H-indol-on,
(±)-3-(5-Chlor-2-hydroxyphenyl)-1,3-dihydro-6-iod-2H-indol-on,
(±)-3-(5-Chlor-2-hydroxyphenyl)-1,3-dihydro-6-(trifluormethyl)-2H-indol-2-on,
(±)-3-(5-Chlor-2-methoxyphenyl)-1,3-dihydro-3-hydroxy-6-(trifluormethyl)-2H-indol-2-on,
(±)-3-(5-Chlor-2-methoxyphenyl)-1,3-dihydro-3-hydroxy-6-(trifluormethyl)-2H-indol-2-on,
(-)-3-(5-Chlor-2-methoxyphenyl)-1,3-dihydro-3-hydroxy-6-(trifluormethyl)-2H-indol-2-on,
(±)-3-(5-Chlor-2-methoxyphenyl)-1,3-dihydro-3-hydroxy-6-iod-2H-indol-2-on,
(±)-3-(5-Chlor-2-hydroxyphenyl)-1,3-dihydro-3-hydroxy-6-(trifluormethyl)-2H-indol-2-on,
(+)-3-(5-Chlor-2-hydroxyphenyl)-1,3-dihydro-3-hydroxy-6-(trifluormethyl)-2H-indol-2-on,
(-)-3-(5-Chlor-2-hydroxyphenyl)-1,3-dihydro-3-hydroxy-6-(trifluormethyl)-2H-indol-2-on,
(±)-3-(5-Chlor-2-hydroxyphenyl)-1,3-dihydro-3-hydroxy-6-iod-2H-indol-2-on,
(±)-3-(5-Chlor-2-methoxyphenyl)-1,3-dihydro-3-fluor-6-(trifluormethyl)-2H-indol-2-on,
(3S)-(+)-(5-Chlor-2-methoxyphenyl)-1,3-dihydro-3-fluor-6-(trifluormethyl)-2H-indol-2-on,
(3R)-(-)-(5-Chlor-2-methoxyphenyl)-1,3-dihydro-3-fluor-6-(trifluormethyl)-2H-indol-2-on,
(±)-3-(5-Chlor-2-methoxyphenyl)-1,3-dihydro-3-fluor-6-phenyl-2H-indol-2-on, oder
(±)-3-(5-Chlor-2-methoxyphenyl)-1,3-dihydro-3-fluor-6-iod-2H-indol-2-on,
oder ein pharmazeutisch akzeptables Additionssalz davon ist.

11. Verwendung gemäß Anspruch 9, worin das 3-substituierte Oxindol-Derivat
(±)-3-(5-Chlor-2-methoxypheny)-1,3-dihydro-3-fluor-6-(trifluormethyl)-2H-indol-2-on,
(3S)-(+)-(5-Chlor-2-methoxyphenyl)-1,3-dihydro-3-fluor-6-(trifluormethyl)-2H-indol-2-on, oder
(3R)-(-)-(5-Chlor-2-methoxyphenyl)-1,3-dihydro-3-fluor-6-(trifluormethyl)-2H-indol-2-on,
oder ein pharmazeutisch akzeptables Additionssalz davon ist.

## Revendications

1. Utilisation d'un dérivé d'oxindole 3-substitué ayant la formule générale I, 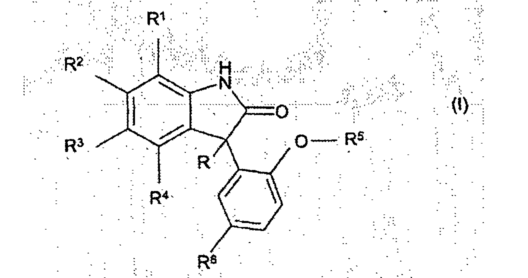 dans laquelle,
R représente de l'hydrogène, un halogène ou un groupe hydroxy,
R¹, R², R³ et R⁴, indépendamment les uns des autres, représentent de l'hydrogène, un halogène, un groupe alkyle, trihalogénométhyle, phényle, *p*-méthyl-phényle ou *p*-trihalogénométhyl-phényle, ou
R¹ et R², R² et R³, ou R³ et R⁴ sont joints ensemble pour former un cycle benzoïque condensé,
R⁵ représente de l'hydrogène ou un groupe alkyle, et
R⁶ représente un halogène ou un groupe trihalogénométhyle,
ou d'un sel d'addition pharmaceutiquement acceptable de celui-ci,
dans laquelle ledit dérivé d'oxindole est capable de moduler le flux ionique à travers les canaux KCNQ,
pour la fabrication d'une composition pharmaceutique, pour le traitement, la prévention ou l'atténuation d'une maladie ou d'un trouble ou d'une affection d'un corps animal vivant, choisi(e) dans le groupe constitué par une douleur, une douleur neuropathique, une céphalée chronique, une douleur centrale, une douleur liée à une neuropathie diabétique, une névralgie postherpétique et à une lésion de nerf périphérique, une dépendance à une drogue, des troubles affectifs, une maladie d'Alzheimer, une anxiété, un mal ou des maladies neurodégénératifs, des déficiences cognitives, un comportement compulsionnel, une démence, une démence liée au VIH, une dépression, une maladie de Huntington, une manie, des troubles cognitifs, une détérioration de la mémoire, des troubles de la mémoire, un dysfonctionnement de la mémoire, des troubles de mobilité, des troubles moteurs, des maladies neurodégénératives, une maladie de Parkinson, des troubles moteurs de type Parkinson, des phobies, une maladie de Pick, une psychose, un trouble bipolaire, une schizophrénie, une lésion médullaire, une cardiomyopathie, une arythmie cardiaque, un syndrome à QT long, un trouble de mobilité, ou un trouble moteur, une myasthénie, une migraine, une céphalée de tension, un trouble intestinal, une maladie inflammatoire, une rectocolite hémorragique, une maladie de Crohn, une maladie de Creutzfeld-Jacobs, une affection ophtalmique, une perte auditive progressive ou tinnitus, une fièvre, une sclérose en plaques, un diabète, ou une croissance tumorale métastatique, une pneumoconiose telle qu'une aluminose, une anthracose, une asbestose, une chalicose, une ptilose, une sidérose, une silicose, un tabagisme et une byssinose ; et une bronchopneumopathie chronique obstructive (COPD) .

2. Utilisation selon la revendication 1, dans laquelle ladite maladie, ledit trouble ou ladite affection est choisi(e) dans le groupe constitué par une douleur, une douleur neuropathique, une céphalée chronique, une douleur centrale, une douleur liée à une neuropathie diabétique, à une névralgie postherpétique et à une lésion de nerf périphérique.

3. Utilisation selon la revendication 1, dans laquelle
R représente de l'hydrogène, un halogène ou un groupe hydroxy,
R¹, R², R³ et R⁴, indépendamment les uns des autres, représentent de l'hydrogène, un halogène, un groupe alkyle, trihalogénométhyle, phényle, *p*-méthyl-phényle
ou *p*-trihalogénométhyl-phényle, et lorsque R¹ et R⁴ sont de l'hydrogène, alors R² ou R³ est un groupe phényle, *p*-méthyl-phényle ou *p*-trihalogénométhyl-phényle ; ou
R¹ et R², R² et R³, ou R³ et R⁴ sont joints ensemble pour former un cycle benzoïque condensé,
R⁵ représente de l'hydrogène ou un groupe alkyle, et
R⁶ représente un halogène ou un groupe trihalogénométhyle.

4. Utilisation selon la revendication 1, dans laquelle
R¹, R³ et R⁴ représentent de l'hydrogène, et
R² représente un halogène, un groupe trihalogénométhyle ou phényle.

5. Utilisation selon la revendication 1, dans laquelle R¹ et R², R² et R³, ou R³ et R⁴ sont joints ensemble pour former un cycle benzoïque condensé.

6. Utilisation selon l'une quelconque des revendications 1 à 4, dans laquelle R⁵ est de l'hydrogène ou un groupe méthyle.

7. Utilisation selon l'une quelconque des revendications 1 à 5, dans laquelle R⁶ est du chlore.

8. Utilisation selon la revendication 1, dans laquelle le dérivé d'oxindole 3-substitué pour l'utilisation selon l'invention est
(+)-3-(5-chloro-2-méthoxyphényl)-1,3-dihydro-6-(trifluorométhyl)-2H-indol-2-one;
(-)-3-(5-chloro-2-méthoxyphényl)-1,3-dihydro-6-(trifluorométhyl)-2H-indol-2-one;
(±)-3-(5-chloro-2-méthoxyphényl)-1,3-dihydro-6-(4-méthylphényl)-2H-indol-2-one;
(±)-3-(5-chloro-2-hydroxyphényl)-1,3-dihydro-2H-indol-one;
(±)-3-(5-chloro-2-hydroxyphényl)-4,6-dichloro-1,3-dihydro-2H-indol-one;
(±)-3-(5-chloro-2-hydroxyphényl)-1,3-dihydro-6-phényl-2H-indol-one;
(±)-3-(5-chloro-2-hydroxyphényl)-1,3-dihydro-6-iodo-2H-indol-one;
(±)-3-(5-chloro-2-hydroxyphényl)-1,3-dihydro-6-(trifluorométhyl)-2H-indol-2-one;
(±)-3-(5-chloro-2-hydroxyphényl)-1,3-dihydro-6-[4-(trifluorométhyl)-phényl]-2H-indol-2-one;
(±)-3-(5-chloro-2-hydroxyphényl)-1,3-dihydro-2H-benz[*e*]indol-2-one;
(±)-3-(5-chloro-2-hydroxyphényl)-1,3-dihydro-2H-benz[*f*]indol-2-one;
(±)-3-(5-chloro-2-hydroxyphényl)-1,3-dihydro-2H-benz[*g*]indol-2-one;
(±)-3-(5-chloro-2-méthoxyphényl)-1,3-dihydro-3-hydroxy-6-(trifluorométhyl)-2H-indol-2-one;
(±)-3-(5-chloro-2-méthoxyphényl)-1,3-dihydro-3-hydroxy-4-(trifluorométhyl)-2H-indol-2-one;
(±)-3-(5-chloro-2-méthoxyphényl)-1,3-dihydro-3-hydroxy-6-(trifluorométhyl)-2H-indol-2-one;
(-)-3-(5-chloro-2-méthoxyphényl)-1,3-dihydro-3-hydroxy-6-(trifluorométhyl)-2H-indol-2-one;
(±)-3-(5-chloro-2-méthoxyphényl)-1,3-dihydro-3-hydroxy-7-(trifluorométhyl)-2H-indol-2-one;
(±)-3-(5-chloro-2-méthoxyphényl)-1,3-dihydro-3-hydroxy-5-bromo-2H-indol-2-one;
(±)-3-(5-chloro-2-méthoxyphényl)-1,3-dihydro-3-hydroxy-6-iodo-2H-indol-2-one;
(±)-3-(5-chloro-2-méthoxyphényl)-4,6-dichloro-1,3-dihydro-3-hydroxy-2H-indol-2-one;
(±)-3-(5-chloro-2-méthoxyphényl)-1,3-dihydro-3-hydroxy-2H-benz[*f*]indol-2-one;
(±)-3-(5-chloro-2-hydroxyphényl)-1,3-dihydro-3-hydroxy-4,6-*bis*-(trifluorométhyl)-2H-indol-2-one;
(±)-3-(5-chloro-2-hydroxyphényl)-1,3-dihydro-3-hydroxy-4-(trifluorométhyl)-2H-indol-2-one;
(±)-3-(5-chloro-2-hydroxyphényl)-1,3-dihydro-3-hydroxy-6-(trifluorométhyl)-2H-indol-2-one;
(+)-3-(5-chloro-2-hydroxyphényl)-1,3-dihydro-3-hydroxy-6-(trifluorométhyl)-2H-indol-2-one;
(-)-3-(5-chloro-2-hydroxyphényl)-1,3-dihydro-3-hydroxy-6-(trifluorométhyl)-2H-indol-2-one;
(±)-3-(5-chloro-2-hydroxyphényl)-1,3-dihydro-3-hydroxy-7-(trifluorométhyl)-2H-indol-2-one;
(±)-3-(5-chloro-2-hydroxyphényl)-4,6-dichloro-1,3-dihydro-3-hydroxy-2H-indol-2-one;
(±)-3-(5-chloro-2-hydroxyphényl)-1,3-dihydro-3-hydroxy-5-bromo-2H-indol-2-one;
(±)-3-(5-chloro-2-hydroxyphényl)-1,3-dihydro-3-hydroxy-6-iodo-2H-indol-2-one;
(±)-1,3-dihydro-3-hydroxy-3-[2-hydroxy-5-(trifluorométhyl)-phényl]-6-(trifluorométhyl)-2H-indol-2-one;
(±)-3-(5-chloro-2-hydroxyphényl)-1,3-dihydro-3-hydroxy-2H-benz[*g*]indol-2-one;
(±)-3-(5-chloro-2-hydroxyphényl)-1,3-dihydro-3-hydroxy-2H-benz[*f*]indol-2-one;
(±)-3-(5-chloro-2-méthoxyphényl)-1,3-dihydro-3-fluoro-6-(trifluorométhyl)-2H-indol-2-one;
(3S)-(+)-(5-chloro-2-méthoxyphényl)-1,3-dihydro-3-fluoro-6-(trifluoromethyl)-2H-indol-2-one;
(3R)-(-)-(5-chloro-2-méthoxyphényl)-1,3-dihydro-3-fluoro-6-(trifluorométhyl)-2H-indol-2-one;
(±)-3-(5-chloro-2-méthoxyphényl)-1,3-dihydro-3-fluoro-7-(trifluorométhyl)-2H-indol-2-one;
(±)-3-(5-chloro-2-méthoxyphényl)-1,3-dihydro-3-fluoro-6-phényl-2H-indol-2-one;
(±)-3-(5-chloro-2-méthoxyphényl)-1,3-dihydro-3-fluoro-6-iodo-2H-indol-2-one;
(±)-3-(5-chloro-2-méthoxyphényl)-1,3-dihydro-3-fluoro-5-méthyl-2H-indol-2-one;
(±)-3-(5-chloro-2-méthoxyphényl)-1,3-dihydro-3-fluoro-5-bromo-2H-indol-2-one;
(±)-3-(5-chloro-2-méthoxyphényl)-1,3-dihydro-3-fluoro-4,6-*bis*-(trifluorométhyl)-2H-indol-2-one; ou
(±)-3-(5-chloro-2-hydroxyphényl)-1,3-dihydro-3-fluoro-7-(trifluorométhyl)-2H-indol-2-one;
ou un sel d'addition pharmaceutiquement acceptable de celui-ci.

9. Utilisation selon la revendication 1, dans laquelle
R représente de l'hydrogène, un halogène ou un groupe hydroxy ;
R¹, R³ et R⁴ représentent de l'hydrogène ;
R² représente un halogène, un groupe trihalogénométhyle ou phényle ;
R⁵ représente de l'hydrogène ou un groupe méthyle ; et
R⁶ représente du chlore.

10. Utilisation selon la revendication 9, dans laquelle le dérivé d'oxindole 3-substitué est
(+)-3-(5-chloro-2-méthoxyphényl)-1,3-dihydro-6-(tirfluorométhyl)-2H-indol-2-one;
(-)-3-(5-chloro-2-méthoxyphényl)-1,3-dihydro-6-(trifluorométhyl)-2H-indol-2-one;
(±)-3-(5-chloro-2-hydroxyphényl)-1,3-dihydro-6-phényl-2H-indol-one;
(±)-3-(5-chloro-2-hydroxyphényl)-1,3-dihydro-6-iodo-2H-indol-one;
(±)-3-(5-chloro-2-hydroxyphényl)-1,3-dihydro-6-(trifluorométhyl)-2H-indol-2-one;
(±)-3-(5-chloro-2-methoxyphényl)-1,3-dihydro-3-hydroxy-6-(trifluorométhyl)-2H-indol-2-one;
(±)-3-(5-chloro-2-méthoxyphényl)-1,3-dihydro-3-hydroxy-6-(trifluorométhyl)-2H-indol-2-one;
(-)-3-(5-chloro-2-méthoxyphényl)-1,3-dihydro-3-hydroxy-6-(trifluorométhyl)-2H-indol-2-one;
(±)-3-(5-chloro-2-méthoxyphényl)-1,3-dihydro-3-hydroxy-6-iodo-2H-indol-2-one;
(±)-3-(5-chloro-2-hydroxyphényl)-1,3-dihydro-3-hydroxy-6-(trifluorométhyl)-2H-indol-2-one;
(+)-3-(5-chloro-2-hydroxyphényl)-1,3-dihydro-3-hydroxy-6-(trifluorométhyl)-2H-indol-2-one;
(-)-3-(5-chloro-2-hydroxyphényl)-1,3-dihydro-3-hydroxy-6-(trifluorométhyl)-2H-indol-2-one;
(±)-3-(5-chloro-2-hydroxyphényl)-1,3-dihydro-3-hydroxy-6-iodo-2H-indol-2-one;
(±)-3-(5-chloro-2-méthoxyphényl)-1,3-dihydro-3-fluoro-6-(trifluorométhyl)-2H-indol-2-one;
(3S)-(+)-(5-chloro-2-méthoxyphényl)-1,3-dihydro-3-fluoro-6-(trifluorométhyl)-2H-indol-2-one;
(3R)-(-)-(5-chloro-2-méthoxyphényl)-1,3-dihydro-3-fluoro-6-(trifluorométhyl)-2H-indol-2-one;
(±)-3-(5-chloro-2-méthoxyphényl)-1,3-dihydro-3-fluoro-6-phenyl-2H-indol-2-one;ou
(±)-3-(5-chloro-2-méthoxyphényl)-1,3-dihydro-3-fluoro-6-iodo-2H-indol-2-one;
ou un sel d'addition pharmaceutiquement acceptable de celui-ci.

11. Utilisation selon la revendication 9, dans laquelle le dérivé d'oxindole 3-substitué est
(±)-3-(5-chloro-2-méthoxyphényl)-1,3-dihydro-3-fluoro-6-(trifluorométhyl)-2H-indol-2-one;
(3S)-(+)-(5-chlor-2-méthoxyphényl)-1,3-dihydro-3-fluoro-6-(trifluorométhyl)-2H-indol-2-one; ou
(3R)-(-)-(5-chloro-2-méthoxyphényl)-1,3-dihydro-3-fluora-6-(trifluorométhyl)-2H--indol-2-one;
ou un sel d'addition pharmaceutiquement acceptable de celui-ci.
